# EUROPÄISCHE PATENTSCHRIFT

(11) **EP 3 586 735 B1**
(45) Veröffentlichungstag und Bekanntmachung des Hinweises auf die Patenterteilung: **04.08.2021**
(21) Anmeldenummer: 19179982.4
(22) Anmeldetag: 13.06.2019
(51) Int. Cl.: A61B 5/274

(54) **OBERFLÄCHENELEKTRODE MIT HALTERUNG FÜR MAGNETSENSOR**
SURFACE ELECTRODE WITH HOLDER FOR MAGNETIC SENSOR
ÉLECTRODE DE SURFACE POURVUE DE SUPPORT POUR UN CAPTEUR MAGNÉTIQUE

(30) Priorität: 29.06.2018 DE 102018115812
(43) Veröffentlichungstag der Anmeldung: 01.01.2020
(73) Patentinhaber: Vanguard AG, 12623 Berlin (DE)
(72) Erfinder: Dr. Pacholik, Alexander, 12203 Berlin (DE); Gutzmer, Thomas, 13599 Berlin (DE); Dr.Bilz, Martin, 14552 Michendorf (DE); Dr. Thielecke, Hagen, 15366 Neuenhagen (DE); Kühner, Ralf, 70567 Stuttgart (DE)
(74) Vertreter: LS-MP von Puttkamer Berngruber Loth Spuhler

(56) Entgegenhaltungen:
- US-A1- 2013 072 870
- US-A1- 2016 302 725

## Beschreibung

Die Erfindung betrifft eine Halterung zur lösbaren Aufnahme eines der Übertragung und/oder des Empfangs elektrischer Ströme und/oder Signale von der Hautoberfläche eines Lebewesens oder von einem Gegenstand im Körper des Lebewesens dienenden Sensors sowie eine Sensoranordnung, die zur Verwendung in einem System zur Bestimmung eines Körpers in einem Lebewesen dient nach dem Anspruch 1 und den Ansprüchen 16 und 21.

Die Erfindung bezieht sich auf - auch im deutschen Sprachgebrauch so bezeichnete - sog. Reference Patches, also eine Art Einwegpflaster für den Gebrauch von wiederverwendbaren Sensoranordnungen zur Verwendung in Systemen zur Bestimmung, insbesondere Ortung, eines Körpers in einem Lebewesen, insbesondere in einem menschlichen Körper, wie auch zur Bestimmung einer Lageveränderung eines Lebewesens, insbesondere einer Person, etwa während einer medizinischen Untersuchung oder Operation. Reference Patches umfassen Oberflächenelektroden mit einer Halterung für in diese Halterung einzusetzende wiederverwendbare Sensoren zur Verwendung in einem solchen System. Reference Patches sind damit in der Regel für den einmaligen Gebrauch vorgesehen, während die in ihnen aufgenommenen Sensoren, die mit einem System zur Bestimmung eines Körpers in einem Lebewesen in Verbindung stehen, also in der Regel mit einem Informations- und Darstellungssystem, zum mehrfachen Gebrauch bestimmt sind.

Sensoren im Sinne der vorliegenden Erfindung sind insbesondere Referenzsensoren. Sie sind hierauf aber nicht beschränkt. Referenzsensoren sind Medizinprodukte, die der Übertragung elektrischer Ströme und/oder der Übertragung sonstiger Signale, insbesondere von der Hautoberfläche eines Lebewesens, insbesondere eines menschliches Körpers, dienen.

Reference Patches bestehen in der Regel aus einem leitenden Elektrodenmaterial, einem selbstklebenden Haftgel, einer Isolierung sowie aus einem elektrischen Anschluss.

Der Einsatz von Referenzsensoren ist insbesondere bei der Elektrophysiologie vorgesehen, um zusammen mit einem Informations- und Darstellungssystem, insbesondere in der Ausgestaltung einer Auswerteeinheit, die 3D-Positionsbestimmung von Medizinprodukten, z.B. Kathetern, während einer Elektrophysiologie-Untersuchung zu ermöglichen.

Dazu werden Referenzsensoren mittels der sog. Reference Patches auf der unverletzten Haut eines Patienten befestigt. Sie werden hierbei dazu genutzt, elektrische Ströme und/oder Signale durch den Patienten zu leiten und/oder zu empfangen, die mittelbar oder unmittelbar von einem Medizinprodukt im Patienten ausgehen. Physikalisch kann dies auf verschiedene Weise geschehen, z.B. durch die Messung und Bestimmung der Stärke eines Magnetfelds, das im Zusammenhang mit dem Medizinprodukt oder mit der Position des Körpers steht.

Ein wesentliches Einsatzgebiet für die D-Positionsbestimmung bzw. -navigation sind Herzdiagnostik- und Ablationskatheter. Die Referenzsensoren ermöglichen u.a. die nicht-fluoroskopische Navigation derartiger Medizinprodukte, insbesondere Katheter, oder etwa die Feststellung einer Lageveränderung des Körpers des Patienten während einer medizinischen Untersuchung oder Operation.

### Stand der Technik:

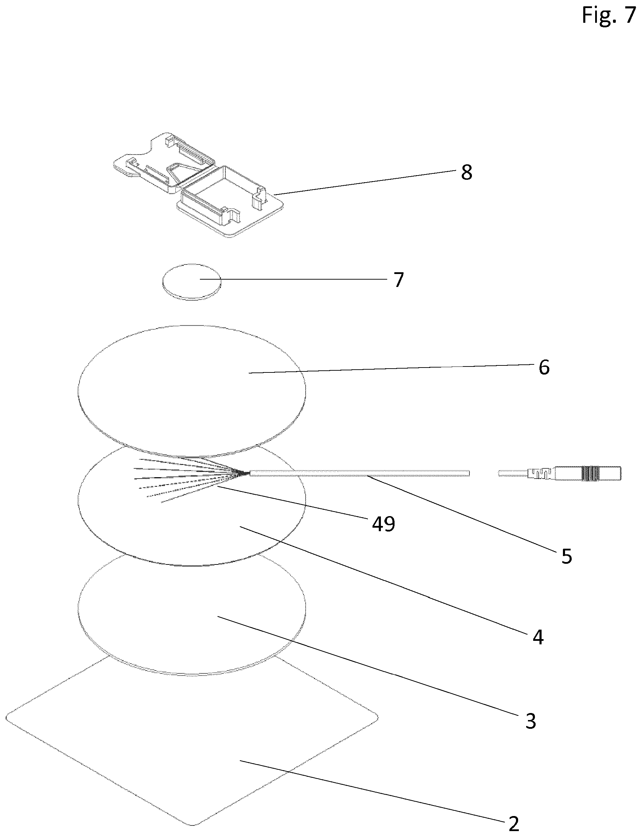

Im Stand der Technik sind verschiedene Reference Patches mit Oberflächenelektroden als Einweg-Produkte zur Verwendung in einem Geräte-Mapping-System zur Abbildung der Lokalisation eines Gerätes im Körper eines Patienten bekannt. Ebenso sind damit einhergehend Pflaster- und Sensoranordnungen zur Verwendung in einem solchen Geräte-Mapping-System bekannt. US 2016/0302725 A1 zeigt eine Pflaster- und Sensoranordnung mit einer Halterung zur lösbaren Aufnahme eines Sensors, wobei die Halterung einen Aufnahmeraum mit einem Boden, einer auf dem Boden angeordneten Wandung mit einer Öffnung und einem Clipverschluss aufweist. Als weiteres Beispiel derartiger Systeme wird auf die europäischen Patentanmeldungen EP 2 389 860 A1 und EP 2 520 223 A1 verwiesen. Diese zeigen u.a. Einweg-Pflasteranordnungen umfassend eine Klebeschicht, mit der die Pflasteranordnung am Körper des Patienten festgeklebt werden kann, eine Elektrodenschicht, eine auf der Elektrodenschicht angeordnete Schaumstoffschicht und ein sog. Eingriffselement für den lösbaren Eingriff eines Abschnitts eines Gehäuses einer wiederverwendbaren Sensorkabelanordnung; die Klebeschicht ist eine Hydrogel-Schicht; die Elektrodenschicht ist auf der klebenden Hydrogel-Schicht angeordnet; weiterhin ist eine Schaumstoffschicht vorgesehen, die eine Vielzahl von Vertiefungen zur Aufnahme eines Sensorkabels der wiederverwendbaren Sensorkabelanordnung aufweist. Der Aufbau eines in diesen Druckschriften genannten Einwegpflasters ist jeweils in den Fig. 7 und 8 zeichnerisch dargestellt. Solche Einwegpflaster zur Aufnahme einer wiederverwendbaren Sensoranordnung zur Verwendung in Systemen zur Ortung und Abbildung medizinischer Vorrichtungen werden auch im Geschäftsverkehr verwendet. Diese Reference Patches besitzen eine Einschubhalter, über den der Magnetsensor während des Eingriffs gehalten wird; weiter ist ein Schaumstoffpolster zur Dämpfung um diesen herum vorgesehen.

In der Praxis sind mit dieser technischen Lösung jedoch Nachteile verbunden.

Bei der bekannten Lösung wird dieser Magnetfeldsensor durch ein zweites Halteelement der Einschubhalterung gehalten. Wenn über das Kabel unter einem bestimmten Winkelbereich Zug auf den Magnetfeldsensor einwirkt, ist das zweite Halteelement wirkungslos. So kann auch das Sensorelement aus der Einschubhalterung gelöst werden. Nachteilig ist daran, dass sich der Magnetsensor auch schon bei geringen Bewegungen des Patienten oder des Kabels des Magnetsensors aus der Halterung lösen kann und der sedierte und vorbereitete Patient ggf. zusätzlich bewegt werden muss für eine erneute Kontaktierung des Magnetsensors. Weiterhin ist nachteilig, dass das zusätzliche Schaumstoffpolster mit der eine Vielzahl von Vertiefungen aufweisenden, eingeprägten Struktur zusätzliche Artefakte und störende Bildüberlagerungen auf den Darstellungssystemen, insbesondere Röntgenkontrollen, verursacht.

Ausgehend von diesen Nachteilen des Standes der Technik stellt sich der vorliegenden Erfindung mithin das technische Problem, d.h. die sog. Aufgabe, sog. Reference Patches mit einer Oberflächenelektrode und einer Halterung für Sensoren vorzusehen, die diese Nachteile sicher vermeiden. Weiterhin ist es ein Ziel, diese Reference Patches kostengünstig als Einweg-Produkte zum hygienischen Einsatz bei diagnostischen und weiteren medizinischen Eingriffsmaßnahmen zur Verfügung zu stellen. Insbesondere sollen diese sog. Reference Patches für den Patienten wenig spürbar und wieder gut ablösbar sein.

Ein besonderes Ziel ist dabei weiterhin, eine sichere und wieder lösbare Aufnahme des Referenzsensors in der Halterung zu ermöglichen, sodass auch während unbeabsichtigter Bewegung des Patienten während der medizinischen Untersuchung eine gesicherte Datenübertragung ermöglicht wird; insbesondere soll ein Verrutschen und ein Sich-Bewegen des Referenzsensors in der Halterung sicher vermieden werden.

Trotz dieser sicheren Aufnahme des Referenzsensors in der Halterung soll weiter gewährleistet sein, dass nach Beendigung der medizinischen Untersuchung der Referenzsensor ohne Beschädigung und auf leichte Weise aus der Halterung wieder entfernt werden kann und danach die Reference Patches von dem Körper des Patienten abgezogen werden können.

Störende Artefakte und Bildüberlagerungen auf den Darstellungssystemen sollen vermieden werden.

Zudem ist es ein Ziel der Erfindung, diese sog. Reference Patches so auszugestalten, dass sie mit zahlreichen auf dem Markt befindlichen Systemen für die Durchführung nicht-fluoroskopischer Positionsbestimmung bzw. Navigation von medizinischen Produkten, insbesondere Kathetern, kompatibel sind.

Diese verschiedenen Aspekte des technischen Problems werden gelöst mit einer Halterung nach Anspruch 1 und mit einer Sensoranordnung nach Anspruch 16 sowie mit einer Verwendung nach Anspruch 21. Bevorzugte Ausführungsbeispiele sind in den jeweiligen Unteransprüchen offenbart.

### Kurzbeschreibung der Erfindung:

Die Erfindung stellt eine Halterung als Klapphalterung für Sensoren, insbesondere Magnetsensoren und im Zusammenwirken mit Oberflächenelektroden zur Verfügung, wobei sie auf ein störendes Schaumstoffpolster verzichtet. Im Gegensatz zu dem vorbeschriebenen Stand der Technik besteht die Neuerung der Erfindung in dem Haltemechanismus für den Sensor und in dem Verzicht auf eine visuelle unter Röntgenstrahlen störende Schaumstoffschicht.

Soweit im Rahmen der vorliegenden Beschreibung von Sensoren die Rede ist, handelt es sich bevorzugt um Magnetsensoren. Es können dabei auch insbesondere magnetoelastische Sensoren zum Einsatz kommen. Ebenso können sie Impulsgeber umfassen

Im Gegensatz zu dem beschriebenen Stand der Technik erfolgt die Kontaktierung des Sensors in dem die Klapphalterung enthaltenden sog. Reference Patch nicht durch das Einschieben in diese Halterung, sondern aufgrund der konstruktiven Ausgestaltung der Halterung durch das Einlegen des Sensors in einen Aufnahmeraum eines Halterunterteils und durch Arretieren des Deckels auf dem Halterunterteil, wodurch eine sichere Fixierung des Sensors in der Halterung erfolgt. Nach dem Eingriff am Patienten kann der Sensor durch einfaches Öffnen wieder entnommen werden. Die Positionierung des Sensors in der Halterung ist von der Vorbereitung des Patienten für den Eingriff bis zum Ende des Eingriffs sicher gestellt.

Die sog. Reference Patches verfügen damit über die Hauptbestandteile einer Elektrode, eines Isolators, eines Haftgels sowie der Halterung und - wenn der Sensor noch kabelgeführt ist - des Anschlusskabels mit Buchse. Die Elektroden weisen ein leitfähiges, mit Carbon versetztes Polymer auf. Sie können etwa einen Durchmesser von 80 mm aufweisen, sind aber nicht auf diese Größenordnung und die Form etwa eines Kreises beschränkt.

Die Rückseite der Elektrode ist mit einem dielektrischen Material versehen, was als Textil bereitgestellt wird und was etwa denselben Durchmesser wie die Elektrode aufweist. Das Textil kann ein Polyestergewebe oder ein Baumwollgewebe sein. Vorzugsweise ist es selbstklebend. Statt eines Gewebes kann auch ein Gewirk, eine sonstige Gitterstruktur, ein Raschel-Produkt eingesetzt werden. Auch diese Alternativen werden im Sinne der vorliegenden Erfindung als Gewebe bezeichnet. Das Gewebe ist jedoch kein Schaumstoff.

Auf der Oberseite des dielektrischen Materials befindet sich eine Halterung aus Kunststoff für die Aufnahme des Magnetsensors. Vorzugsweise ist die Halterung mittig angeordnet. Bei einer kabelführenden Ausgestaltung ist die Elektrode mit einem Kabel konfektioniert, an dessen Ende sich eine Buchse für die Aufnahme von elektrischen Stiften befinden kann. An diese wiederum ist ein Verlängerungskabel mit einer bestimmten Länge angebracht. Alle Buchsen und Stecker und Kabel besitzen vorzugsweise einen Knickschutz.

Das Haftgel kann ein marktübliches Hydrogel sein.

Die Halterung kann aus jeglichem geeigneten Material bestehen. Insbesondere wird ein Kunststoff in Form eines Polycarbonats gut geeignet sein.

Bei dem Kleber kann es sich um jeglichen geeigneten handelsüblichen Kleber handeln.

Diese vorstehende Anordnung ist vorzugsweise auf einer Trägerfolie angeordnet, die etwa aus Polyethylen bestehen kann und einseitig silikonisiert ist.

### Detailliertere Beschreibung:

Betrachtet man den räumlichen Aufbau dieser solchen Reference Patches von unten nach oben, so umfasst dieses vorzugsweise zunächst die vorerwähnte Trägerfolie, die aus Polyethylen, einseitig silikonisiert, bestehen kann. Es folgen das Hydrogel und dann die Elektrode. Die eigentliche Elektrode umfasst das auf der Trägerfolie anordenbare Hydrogel, das nach Abziehen der Trägerfolie auf die Haut des Patienten appliziert wird. Auf dem Hydrogel wiederum ist also die Elektrode angeordnet, die bevorzugt aus einem leitfähigen, mit Carbon versetzten Polymer besteht. Auf die Oberseite dieser Elektrode wird das elektrische Kabel appliziert, das vorzugsweise mit weichem PVC ummantelt ist; insbesondere ist eine Carbon-Polyesterfaser mit Weich-PVC-Ummantelung vorteilhaft.

Auf diese vorerwähnte Gesamtanordnung wird der Isolator angeordnet, bei dem es sich, wie dargestellt, um ein Polyestergewebe oder um ein Baumwollgewebe handeln kann. Vorzugsweise ist dieses Gewebe an seiner Unterseite selbstklebend. Auf der Oberseite dieses Gewebes wird ein Kleber oder ein Klebepunkt aufgetragen, auf den dann die Halterung geklebt wird, die der Aufnahme des (Magnet-)Sensors dient.

Die Halterung dient der lösbaren Aufnahme eines Sensors. Der Sensor empfängt oder überträgt elektrische Ströme und/oder sonstige Signale von der Hautoberfläche eines Patienten bzw. von einem im Körper des Lebewesens befindlichen Objekt.

Die Halterung weist vorzugsweise ein Halterunterteil und einen Deckel auf. Das Halterunterteil und der Deckel sind durch ein Verbindungselement derart miteinander verbunden, dass der Deckel zum Halterunterteil bewegbar, d.h. klappbar, ist und dort mit noch näher zu beschreibenden Mitteln fixierbar ist.

In einem geöffneten Ausgangszustand sind der Deckel und das Halterunterteil also in einer horizontalen Ebene.

Vorzugsweise ist die Halterung materialeinheitlich ausgestaltet. Weiter vorteilhaft ist es, wenn sie einstückig ausgestaltet ist.

Das Halterunterteil und der Deckel können dabei eine in etwa gleiche Grundfläche aufweisen. Sie sind im offenen Zustand betrachtet durch ein Verbindungselement miteinander verbunden, das jeweils eine Stirnseite des Halterunterteils und des Deckels miteinander verbindet.

Bei dem distal zum Boden des Halterunterteils angeordneten Verbindungselement kann es sich hierbei um ein herkömmliches Filmscharnier handeln. Es ist aber auch jegliche andere gelenkige Verbindungsstruktur möglich. Vorteilhaft ist die Ausgestaltung als Bandscharnier, bei dem ein flexibles, dünnwandiges Gelenkrelais zwischen den beiden zu verbindenden Teilen des Deckels und des Halterunterteils vorhanden ist. Das Verbindungselement ist so gewählt, dass es die erforderliche hohe Biegewechselfestigkeit aufweist.

Weiter ist es möglich, das Verbindungselement etwa als Schnappscharnier auszugestalten, bei dem zwei stabile Endlagen ermöglicht werden, nämlich der geöffnete und der geschlossene Zustand von Deckel und Halterunterteil.

Das Halterunterteil enthält den eigentlichen Aufnahmeraum zur Aufnahme des Sensors. Dieser Aufnahmeraum wird durch einen Boden des Halterunterteils gebildet, von dem ausgehend eine den Aufnahmeraum umgrenzende Wandung angeordnet ist. Die Wandung ist unter Berücksichtigung der üblichen geometrischen Ausgestaltung derartiger Sensoren so aufgebaut, dass sie wenigstens über drei Seiten verfügt, die in etwa U-förmig zueinander angeordnet sind. In dieser U-Form können die jeweiligen Seiten über Ecken miteinander verbunden sein. Die Wandung kann aber auch aus drei nicht miteinander verbundenen Seitenteilen bestehen, bei denen also die jeweilige Eckverbindung zueinander fehlt.

Bei einer rechteckigen oder quadratischen Grundform des Aufnahmeraums weist die vierte Seite eine Öffnung auf, die noch weiter unten näher beschrieben wird. Diese Öffnung dient der Aufnahme etwa des Kabels des Sensors, über das dieser mit dem Informations- und Darstellungssystem verbunden ist. Weiter kann diese Öffnung so ausgestaltet sein, dass sie eine Kabelschutzumhüllung (Knickschutz) aufnimmt.

Von der vorbeschriebenen Wandung und dem Boden des Halterunterteils abgesehen, ist der Aufnahmeraum zur Aufnahme des Sensors nach oben hin offen, weil, wie noch näher erläutert werden wird, der Sensor nicht durch die Öffnung an der einen Seite der Wandung durchgeschoben werden kann, sondern von oben in den Aufnahmeraum gelegt wird.

Diese Öffnung an der einen Seite der Wandung wird durch wenigstens einen Steg in ihrer Breite begrenzt. Vorzugsweise sind zwei Stege vorhanden, die von den beiden offenen Stirnseiten der U-förmigen Wandung ausgehen. Der wenigstens eine Steg bzw. die beiden Stege ist bzw. sind so ausgestaltet, dass er bzw. sie den Abstand der beiden gegenüberliegenden Schenkel der U-förmigen Wandung verkürzt bzw. verkürzen. Hierzu ist/sind sie vorzugsweise etwa als rechtswinklige Anbauteil(e) ausgestaltet, wobei ein Schenkel des wenigstens einen Steges in proximaler Weise vorsteht. Beim Einsatz von zwei Stegen entsteht hierdurch eine Verengung der Öffnung, die man etwa als flaschenförmigen Hals umschreiben könnte.

Dieser mit einer derartigen flaschenhalsförmigen Verengung vorgesehene U-förmige Aufnahmeraum enthält weiter wenigstens einen Clipverschluss, der beispielsweise als Rastnase oder Rasthaken oder dergleichen ausgestaltet sein kann. Dieser Clipverschluss ist bevorzugt am proximalen Ende der U-förmigen Wandung angeordnet, und zwar bevorzugt auf der oberen Stirnseite der Wandung oder auf der oberen Stirnseite des wenigstens einen Steges.

Dieser Clipverschluss dient im Zusammenspiel mit seinem Gegenstück, das an der Innenseite des Deckels angeordnet ist, dem Verschließen der Halterung und dem Ermöglichen des Wieder-Öffnens der Halterung.

Entsprechend dem durch die Wandung und den Boden ausgestalteten Aufnahmeraum in dem Halterunterteil umfasst die Halterung weiterhin einen Deckel, der zum Verschließen und Wieder-Öffnen des Halterunterteils vorgesehen ist. An der Innenseite des Deckels ist eine wenigstens an drei Seiten angeordnete Wandung vorgesehen, deren (in aufgeklapptem Zustand: obere, horizontal verlaufende) Stirnseite zur Auflage auf der entsprechend oberen, horizontal verlaufenden Stirnseite der Wandung des Halterunterteils ausgestaltet ist.

Durch das Umklappen des Deckels auf das Halterunterteil und das Aneinanderliegen der jeweiligen Stirnseiten der U-förmigen Wandung des Halterunterteils und der Stirnseite der U-förmigen Wandung des Deckels wird mithin ein bis auf die Öffnung an der einen Seite beider Wandungen geschlossener Raum bewerkstelligt, der den Sensor vollständig umgibt. Lediglich durch die Öffnung an der offenen Seite der U-förmigen Ausgestaltung bei der Wandungen tritt das Kabel bzw. der Knickschutz des Kabels des Sensors aus dieser Anordnung heraus.

An der Stirnseite der an der Innenseite des Deckels angeordneten Wandung ist wenigstens ein Gegenstück zu dem wenigstens einen Clipverschluss der Wandung des Halterunterteils angeordnet. Die Anordnung erfolgt dabei vorzugsweise so, dass bei Umklappen des Deckels und bei Arretierung des Deckels auf der Halterunterseite der Halterung das wenigstens eine Gegenstück den wenigstens einen Clipverschluss der Wandung des Halterunterteils übergreift.

Das Halterunterteil und der Deckel sind mithin über den wenigstens einen Clipverschluss sowie das wenigstens eine Gegenstück zu dem Clipverschluss gegeneinander arretierbar und auch wieder zu öffnen. Durch den Clipverschluss und das Gegenstück tritt beim Schließen des Deckels und des Halterunterteils ein hörbares Klickgeräusch auf.

Die Öffnung zur Aufnahme eines Sensorkabels oder einer Kabelschutzumhüllung (Knickschutz) des Sensors ist an der proximalen Seite des Halterunterteils angeordnet. Proximal bezeichnet dabei die Seite der Halterung, an der das Sensorkabel aufgenommen ist. Die flaschenhalsförmige Verengung der Öffnung der Wandung des Halterunterteils erfolgt dergestalt, dass der Sensor nur durch Einlegen in den Aufnahmeraum angeordnet werden kann, d.h. ein seitliches oder schrägseitlich erfolgendes Einschieben des Sensors durch die Öffnung ist nicht beabsichtigt, da dies auch zu einem ungewollten Herausziehen des Sensors aus dem Aufnahmeraum des Halterunterteils führen kann, was vermieden werden soll.

Vorzugsweise ist der wenigstens eine Clipverschluss an der oberen Stirnseite der Wandung oder der oberen Stirnseite des Steges angeordnet und steht hiervon axial oder radial ab.

Dies gilt auch für das spiegelbildliche wenigstens eine Gegenstück, das von der oberen Stirnseite der Wandung des Deckels axial oder radial absteht und das beim Schließen des Deckels den Clipverschluss der Wandung des Halterunterteils vorzugsweise übergreift.

Der wenigstens eine Clipverschluss der Wandung des Halterunterteils und/oder das vorerwähnte Gegenstück auf Seiten des Deckels kann/können eine Verrundung zur Vermeidung von Spannungsspitzen beim Arretieren und/oder Öffnen des Deckels aufweisen.

In einer bevorzugten Ausführungsform weist die obere, horizontal verlaufende Stirnseite der Wandung einen Rücksprung auf. Dies ermöglicht, dass die Stirnseite der Wandung der Innenseite des Deckels beim Arretieren des Deckels mit dem Halterunterteil auf der oberen Stirnseite des Rücksprungs der Wandung zum Aufliegen kommt. Dadurch wird ein dichtes, gegen seitliches Verschieben geschütztes Umschließen des Aufnahmeraums ermöglicht und der in diesem Aufnahmeraum aufzunehmende Sensor vor Einflüssen geschützt, die seitlich auf die Halterung bzw. auf den Deckel einwirken.

Vorzugsweise können der Boden des Halterunterteils und/oder der Deckel der Halterung wenigstens einen Randbereich aufweisen, der über die Auflagefläche der Wandung radial nach außen gerichtet ist. Dadurch entsteht ein bereichsweiser Überstand der Deckelplatte und/oder des Bodens über den durch beide Wandungen definierten Aufnahmeraum für den Sensor. Dadurch lässt sich das von Hand zu betätigende Schließen bzw. Öffnen der Halterung erleichtern.

Die Platte des Deckels weist vorzugsweise einen eine bewegliche Zunge aufnehmenden Ausschnitt auf. Die bewegliche Zunge ist dadurch federnd gelagert. In einer weiter besonders vorteilhaften Ausgestaltung weist die Zunge eine im geschlossenen Zustand des Halters nach innen gerichtete Verdickung, z.B. einen Wulst, auf. Dieser Wulst liegt im geschlossenen Zustand der Halterung auf der Oberseite des Sensors auf, sodass der Sensor auch gegen vertikale Bewegungen in der geschlossenen Halterung hinreichend geschützt ist. Durch die bewegliche Ausgestaltung der Zunge wird dabei quasi ein sanfter Druck auf die Oberseite des Sensors ausgeübt, sodass gleichwohl ein geringfügiges Bewegungsspiel der Zunge verbleibt und dadurch eine Passgenauigkeit des Sitzes des Sensors in dem Aufnahmeraum ermöglicht wird, die auch ggf. auftretende Herstellungstoleranzen des Gehäuses des aufzunehmenden Sensors ausgleichen kann.

Weiterhin ist es vorteilhaft, wenn der Deckel eine Ausnehmung aufweist, die im geschlossenen Zustand der Halterung an deren proximalen Ende liegt. Hierdurch werden gewisse Ausgleichsbewegungen des Sensorkabels bzw. der Kabelschutzumhüllung (Knickschutz) des Kabels in vertikaler Richtung ermöglicht, was je nach Lokalisation der Anordnung der Oberflächenelektrode mit der vorerwähnten Halterung auf der Haut des Patienten wünschenswert ist. Da sich diese Ausnehmung nur am Deckel befindet, bedeutet dies, dass bei einer solchen Ausgleichbewegung des Sensorkabels bzw. der Kabelschutzumhüllung (Knickschutz) des Sensorkabels der Sensor gegen den Boden des Halterunterteils gedrückt wird, sodass auch im Fall einer solchen Ausgleichsbewegung die Kontaktierung des Sensors in dem Reference Patch aufrechterhalten bleibt und die Kontaktierung des Magnetsensors mit der Elektrode in jeglichem Anwendungszustand gleichbleibend gesichert ist.

Aus dem gesamten Vorstehenden ergibt sich, dass es bevorzugt ist, die Halterung und die Oberflächenelektrode als Einwegprodukt auszugestalten, das für den einmaligen Gebrauch bestimmt ist.

Neben der erfindungsgemäßen Ausgestaltung der vorbeschriebenen Halterung ist die Erfindung auf eine Sensoranordnung gerichtet, die zur Verwendung in einem System zur Bestimmung eines Körpers in einem Lebewesen geeignet und bestimmt ist, und die umfasst eine Halterung, wie sie vorstehend beschrieben wurde, eine Klebeanordnung, einen Isolator aus Polyestergewebe oder Baumwollgewebe und einen wiederverwendbaren (Referenz-)Sensor zur Bestimmung und Bereitstellung von Informationen zu einem Körper in einem Lebewesen an ein Informations- und Darstellungssystem, eine Elektrode sowie ein Hydrogel.

Ferner ist die Erfindung auf eine Verwendung der Halterung in einem Reference Patch gerichtet.

Bei diese Anordnung ist ein besonderer Vorteil, dass diese einen Isolator einsetzt, der schaumstofffrei ausgestaltet ist, sodass die eingangs erwähnten Artefakte und störenden Bildüberlagerungen auf den Röntgenkontrollen nicht auftreten, was bei der Verwendung eines zusätzlichen Schaumstoffpolsters jedoch möglich ist.

Die Ausgestaltung der Sensoranordnung und der Halterung ermöglicht dabei auch ein kabelloses Arbeiten des Sensors (z.B. über Bluetooth) gleichermaßen wie ein Arbeiten mit einem Kabel zusammen mit dem Informations- und Darstellungssystem.

### Beschreibung eines Ausführungsbeispiels:

Nachfolgend wird die vorstehend wiedergegebene Erfindung anhand eines Ausführungsbeispiels weiter erläutert, wobei die Erfindung nicht auf eine Konfiguration gemäß diesem Ausführungsbeispiel beschränkt ist. Dabei zeigen:
Fig. 1: eine Gesamtansicht der Oberflächenelektrode mit geöffneter Halterung;
Fig. 2: eine Darstellung des Einlegens des Sensors in die Halterung;
Fig. 3: eine Darstellung der Schließbewegung des Deckels zur Befestigung des in das Halterunterteil eingelegten Sensors;
Fig. 4: eine Darstellung der Arretierbewegung des Deckels zum Verschließen der Halterung;
Fig. 5: die Darstellung der Öffnungsbewegung des Deckels zum Zwecke der Entfernung des Sensors aus der Halterung;
Fig. 6: die Herausnahmebewegung des Sensors aus der wiedergeöffneten Halterung;
Fig. 7: den Aufbau der Oberflächenelektrode mit Halterung zur Aufnahme des Sensors.

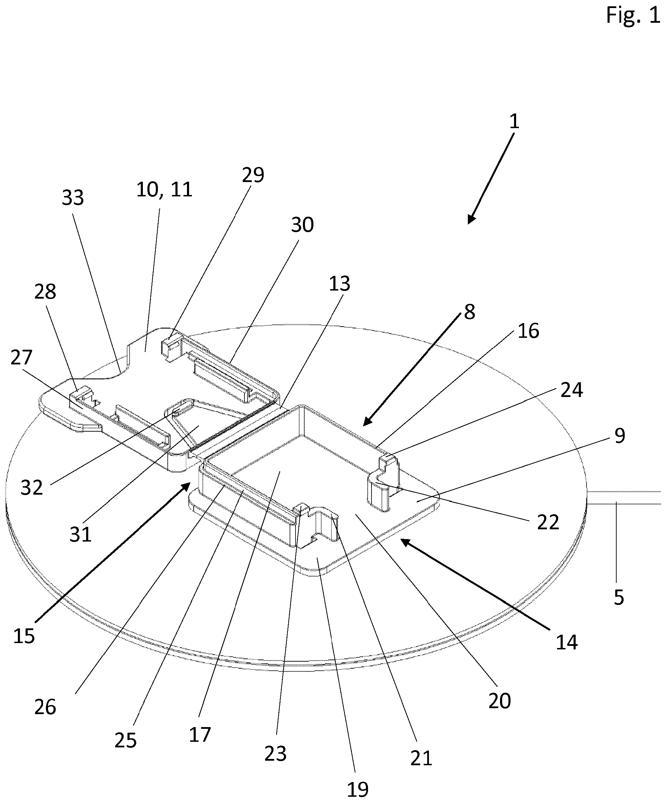

Fig. 1 zeigt den Aufbau und die Anordnung der Halterung auf der Oberflächenelektrode. Die Oberflächenelektrode ist als kreisrundes Teil dargestellt. Die Halterung wird mittels eines (nicht dargestellten) Klebers entweder punktförmig oder flächenförmig auf der Oberseite der gezeigten Oberflächenelektrode, d.h. des Isolators 6, befestigt. Die Halterung ist in geöffnetem Zustand dargestellt.

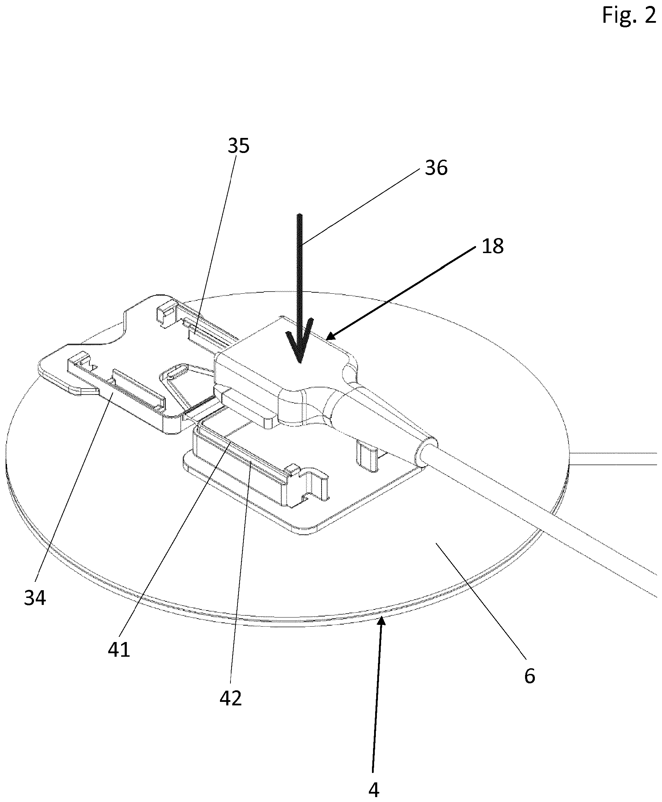

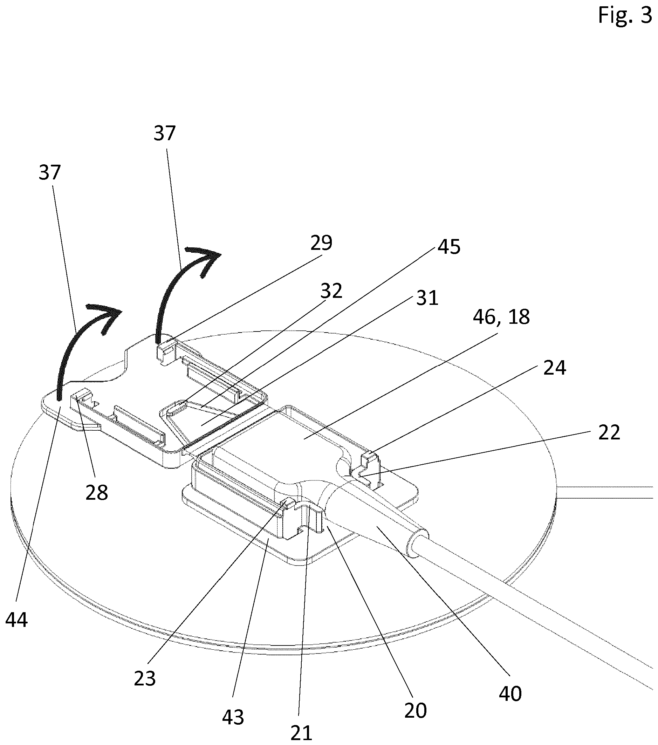

Sie umfasst gemäß Fig. 1 ein Halterunterteil 9 mit einem Aufnahmeraum 17, der der Aufnahme des in Fig. 2, Fig. 3 gezeigten Sensors 18 dient. Der Aufnahmeraum 17 wird ausweislich Fig. 1 aus einem flächig ausgestalteten Boden 19 gebildet, der die Basis für eine dort angeordnete, wenigstens drei Seiten umfassende Wandung 16 ist. Die Wandung 16 ist dabei U-förmig, d.h. an dem proximalen Ende 14 der Halterung 8 sind die Wandung 16 und damit auch der Aufnahmeraum 17 nicht geschlossen. Bei der dreiseitigen Anordnung der Wandung sind die jeweiligen Seiten der Wandung 16 miteinander an ihren jeweiligen Ecken verbunden; möglich wäre aber auch, wenn insoweit offene Ecken vorhanden wären.

Fig. 1 zeigt, dass die Öffnung 20 des Aufnahmeraums 17 durch zwei Stege 21, 22 gebildet wird. Die Stege sind an den proximalen, vertikalen Stirnseiten der Wandung 16 angeordnet und weisen eine in einem Winkel verlaufende Form aus, dergestalt, dass die Öffnung 20 gegenüber dem parallelen Verlauf der Wandungsseiten verengt wird. Diese Verengung lässt sich etwa als flaschenhalsförmige Verengung bezeichnen. Die verengte Öffnung 20 an der proximalen Seite 14 des Halterunterteils 9 dient der Aufnahme des Sensorkabels 5 bzw. der Kabelschutzumhüllung 40 des Sensors 18, wie dies deutlich in Fig"3 dargestellt ist. Die dort gezeigte Ausgestaltung lässt erkennen, dass der durch die Stege 21, 22 geschaffene verengte Abstand der Öffnung 20 so ausgestaltet ist, dass insbesondere eine Knickschutztülle des Kabels gut passend geführt wird. Dadurch, dass die Stege 21, 22 zu einer länglichen Verlängerung des Aufnahmeraums 17 führen, die Wandung 16 also in diesem Bereich verlängert wird, tritt eine verbesserte Führung des Kabels 5 bzw. der Kabelschutzumhüllung 40 ein, was für die Kontaktierung des Sensors in der Referenzelektrode vorteilhaft ist. Durch diese Verlängerung des Aufnahmeraums in Richtung des proximalen Endes der Halterung wird also eine über die durch die Kabelschutzumhüllung 40 ohnehin bereits vorhandene Stabilisierung hinausgehende Lagefixierung ermöglicht.

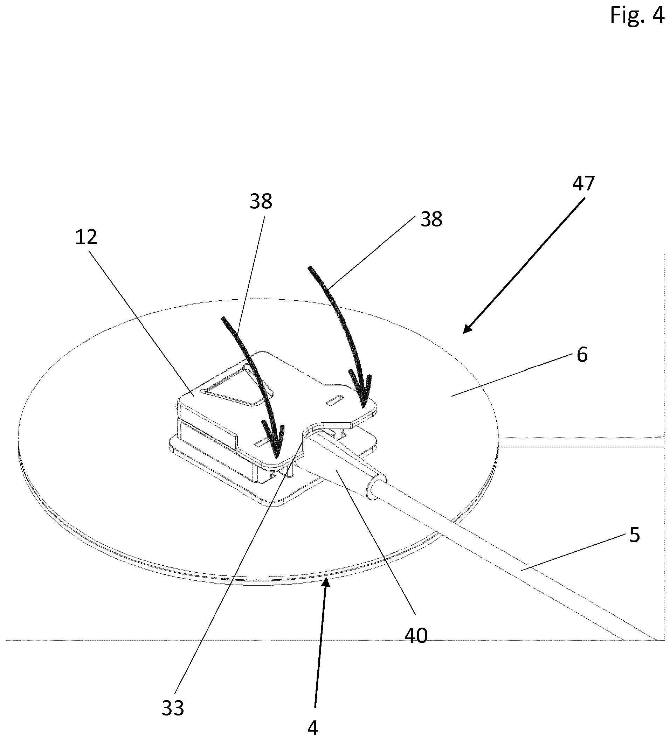

Die vorstehende Ausgestaltung des Aufnahmeraums gemäß Fig. 1, Fig. 2 und Fig. 3 zeigt, dass der Sensor 18 nicht in den Aufnahmeraum 17 durch die Öffnung 20 seitlich eingeschoben werden kann, sondern von oben in den offenen Aufnahmeraum 17 eingesetzt wird, was durch den Pfeil 36 in Fig. 2 zum Ausdruck gebracht wird. Dementsprechend ist der Sensor 18 auch gegen unbeabsichtigtes Herausziehen aus der geschlossenen Halterung durch Passieren der Öffnung 20 gesichert, was den Fig. 3 und 4 entnehmbar ist.

Weiter kann der Fig. 1 sowie Fig. 2 entnommen werden, dass die Wandung 16 an ihrer oberen, horizontal verlaufenden, Stirnseite 41 einen Rücksprung 25 aufweist. Dieser Rücksprung ist in diesem Ausführungsbeispiel alle drei Seiten der Wandung umlaufend, wobei in Fig. 1 dies nur bei der vorderen Seite erkennbar macht. Statt einer umlaufenden Ausgestaltung des Rücksprungs können auch sektionale Rücksprünge verwendet werden. Die Oberseite des Rücksprungs 25 ist mit der Bezugsziffer 26 gekennzeichnet.

In Fig. 1 ist weiterhin als Bestandteil der Halterung 8 ein Deckel 10 dargestellt, der im in Fig. 3, 4, 5 gezeigten Schließzustand das Halterunterteil 9 bedeckt. An der Innenseite 11 des Deckels 10 ist, wie beispielsweise in Fig. 1 gezeigt, eine Wandung 27 angeordnet, die ebenso U-förmig angeordnet ist wie die Wandung 16 des Halterunterteils 9, sodass beim Schließen des Deckels 10 ein passgenaues Aufeinanderliegen der jeweiligen oberen, horizontal verlaufenden Stirnseiten der Wandung 27 und 16 gewährleistet ist. Dabei liegt die obere Stirnseite 30 der Wandung 27 auf der Stirnseite 41 der Wandung 16 des Halterunterteils im Schließzustand auf.

In der in Fig. 1 und Fig. 2 beispielsweise gezeigten Ausführungsform erfolgt die Auflage der Wandung 27 dabei in der Weise, dass die Stirnseite 30 der Wandung 27 der Innenseite 11 des Deckels 10 beim Arretieren des Deckels 10 mit dem Halterunterteil 9 auf der oberen Stirnseite 42 des Rücksprungs 25 der Wandung 16 aufliegt.

Wie z.B. in Fig. 1 und Fig. 3 dargestellt, sind am proximalen Ende der Wandung 16 Clipverschlüsse 23, 24 angeordnet, die von der oberen Stirnseite der Wandung 16 axial nach oben abstehen und dabei (nicht gezeigt) einen nasenartigen, in Richtung proximales Ende der Halterung zeigenden Vorsprung beinhalten. Diese Clipverschlüsse 23 und 24 arbeiten mit entsprechenden Gegenstücken 28 und 29 auf der Seite des Deckels zusammen, die ebenfalls am - im geschlossenen Zustand der Halterung - proximalen Ende des Deckels über die Clipverschlusse 23 und 24 einrasten. Zu diesem Zweck stehen in diesem Ausführungsbeispiel die Gegenstücke 28 und 29 ebenfalls in der Höhe von der Wandung 27 ab. Sie sind dabei an den proximalen Stirnseiten der parallelen Wandflächen 27 angeordnet.

Das Halterunterteil 9 sowie der Deckel 10 sind durch ein am distalen Ende 15 des Halterunterteils 9 angeordnetes Verbindungselement 13 derart miteinander verbunden, dass der Deckel 10 zum Halterunterteil 9 bewegbar ist und das Halterunterteil 9 und der Deckel 10 über die beiden Clipverschlusse 23 und 24 sowie die beiden Gegenstücke 28 und 29 gegeneinander arretierbar sind und auch nach dem Schließzustand wieder geöffnet werden können. Das Verbindungselement ist in dieser Ausführungsform als Filmscharnier ausgestaltet.

Beispielsweise in den Fig. 1 und 3 ist dargestellt, dass der Boden 19 des Halterunterteils wenigstens bereichsweise einen Randbereich 43 bildet, der über die Auflagefläche der Wandung 16 radial nach außen gerichtet ist bzw. vorsteht; bei dem Deckel 10 ist vergleichsweise ein wenigstens bereichsweise ausgestalteter Randbereich 44 vorhanden, der über die Auflagefläche der Wandung 27 radial nach außen gerichtet ist. Dadurch lässt sich die Deckelanordnung bei der Einlegung des Sensors, bei der Fixierung und bei dem Entnehmen des Sensors gut handhaben.

Wie aus Fig. 1 und Fig. 4 ersichtlich ist, weist die Fläche des Deckels 10 eine Ausnehmung 33 auf, die im geschlossenen Zustand der Halterung 8 an deren proximalen Ende liegt. Dies ermöglicht gewisse Ausgleichsbewegungen bei der Positionierung des Sensors bzw. der Kabelschutzumhüllung 40; entsprechend entsteht dadurch auch eine gewisse Bewegungsfreiheit während der Durchführung der Untersuchungen bei den Patienten.

Fig. 2 zeigt parallel zur Wandung 27 verlaufende Fixierungselemente 34 und 35, die beim Arretieren des Deckels auf dem Halterunterteil 9 zu einer passgenauen Fixierung des Sensors 18 in dem Aufnahmeraum 17 führen. Pfeil 36 zeigt die Einlegebewegung des Sensors 18 in den Aufnahmeraum 17.

Fig. 3 lässt mit den Pfeilen 37 die Schließbewegungsrichtung des Deckels 10 bei eingelegtem Sensor 18 erkennen. Weiterhin zeigt die Figur, dass der Deckel 10 bereichsweise einen in die Fläche des Deckels geschnittenen Ausschnitt 45 umfasst, der eine bewegliche Zunge 31 aufnimmt. Diese weist in der gezeigten Ausführungsform eine Verdickung 32 auf, die im Schließzustand der Halterung 8 auf der Oberseite 46 des Sensors 18 zu liegen kommt und damit zu einer auch vertikalen Fixierung des Sensors in dem Aufnahmeraum 17 beiträgt, wobei die bewegliche Ausgestaltung der Zunge 31 eine gewisse vertikale Ausgleichsbewegung ermöglicht. Hierdurch wird insgesamt ein flexibler, nach unten gerichteter Druck des Sensors 18 in den Aufnahmeraum 17 bewerkstelligt, sodass eine permanente Kontaktierung des Sensors mit der Referenzelektrode gewährleistet ist.

Fig. 4 zeigt den Referenzsensor 47 in seinem Zustand unmittelbar vor der Arretierung. Die Arretierung erfolgt mittels eines in Pfeilrichtung 38 erfolgenden Drucks auf die Oberseite 12 des Deckels 10, wodurch der bereits beschriebene Einrastvorgang zwischen den Clipverschlüssen 23 und 24 sowie den Gegenstücken 28 und 29 erfolgt.

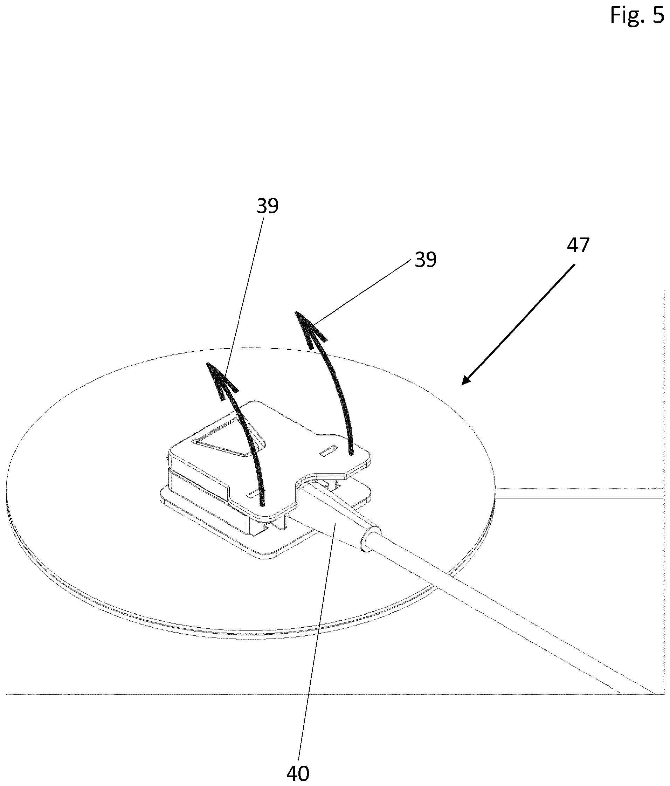

Die Pfeile 39 zeigen in Fig. 5 an, wie die Halterung 8 durch Aufwärtsbewegen des Deckels 10 vom verschlossenen Zustand wieder geöffnet wird.

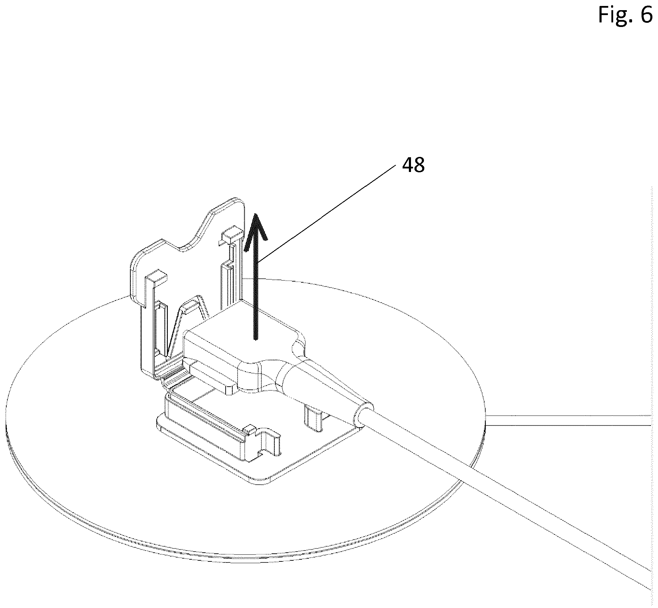

Fig. 6 zeigt die Entnahmebewegung des Sensors 18 aus dem Aufnahmeraum 17 mittels Pfeils 48.

In den Fig. 1 - Fig. 6 ist gemeinsam, dass die Halterung 8 auf der Oberfläche des Isolators 6 angeordnet ist. Die unter dem Isolator 6 angeordnete Elektrode 4 ist angedeutet.

Der Grundaufbau der Oberflächenelektrode mit Halterung kann der Fig. 7 entnommen werden. Sie ist schaumstofffrei ausgestaltet. Sie zeigt von oben betrachtet die erläuterte Halterung 8, die mittels eines Klebers 7, der auf der Oberseite des Isolators 6 angeordnet ist. Der Isolator 6 ist dabei als (Polyester- oder Baumwoll-)Gewebe ausgestaltet. Die Unterseite des Isolators ist selbstklebend ausgestaltet, sodass hierdurch eine feste Verbindung des Isolators 6 mit der Oberseite der Elektrode 4 ermöglicht wird. Zwischen Halterung 8 und Isolator 6 ist keine Schaumstoffschicht angeordnet. Dazwischen liegt das Kabel 5 mit seinem offenen (Kupfer-)Kabelende 49 zur Bewerkstelligung der durch die Anordnung gewünschten Signale/elektrischen Ströme. Die Unterseite der Elektrode 4 ist mit einem Hydrogel beschichtet, das nach Abzug der silikonisierten Trägerfolie 2 auf die Haut des Patienten appliziert wird, wodurch die für die Durchführung der medizinischen Untersuchung notwendige Befestigung der Oberflächenelektrode mit Halterung bewerkstelligt wird.

Aus den Figuren ist auch eine entsprechende Sensoranordnung 47 entnehmbar, die das Reference Patch und den wiederverwendbaren Sensor 18 inkludiert.

### Bezugszeichenliste

- 1: Einweg-Pflasteranordnung
- 2: Trägerfolie
- 3: Hydrogel
- 4: Elektrode
- 5: Sensorkabel
- 6: Isolator
- 7: Klebeanordnung
- 8: Halterung
- 9: Halterunterteil für Sensor
- 10: Deckel
- 11: Deckelinnenseite
- 12: Deckelaußenseite
- 13: Verbindungselement
- 14: proximales Ende Halterung 8
- 15: distales Ende Halterung 8
- 16: Wandung Halterunterteil 9
- 17: Aufnahmeraum für Sensor
- 18: Sensor
- 19: Boden Halterunterteil 9
- 20: Öffnung proximales Ende Halterung 8
- 21: Steg
- 22: Steg
- 23: Clipverschluss
- 24: Clipverschluss
- 25: Rücksprung an Stirnseite Wandung 16
- 26: Oberseite Rücksprung 25
- 27: Wandung Deckel 10
- 28: Gegenstück
- 29: Gegenstück
- 30: Stirnseite Wandung 27
- 31: bewegliche Zunge
- 32: Verdickung Zunge 31
- 33: Ausnehmung
- 34: Fixierungselement
- 35: Fixierungselement
- 36: Pfeil Einsetzrichtung Sensor 18
- 37: Pfeil Schließrichtung Deckel 10
- 38: Pfeil Druckrichtung Deckel 10
- 39: Pfeil Öffnungsrichtung Deckel 10
- 40: Kabelschutzumhüllung
- 41: obere Stirnseite der Wandung 16
- 42: obere Stirnseite des Rücksprungs 25
- 43: Randbereich Boden 19
- 44: Randbereich Innenseite 11 Deckel 10
- 45: Ausschnitt
- 46: Oberseite Sensor 16
- 47: Sensoranordnung
- 48: Pfeil Entnahmerichtung Sensor
- 49: offene Kabelenden zu Kabel 5

## Patentansprüche

1. Halterung (8) zur lösbaren Aufnahme eines der Übertragung und/oder des Empfangs elektrischer Ströme und/oder Signale in Bezug auf einen Körper eines Lebewesens dienenden Sensors (18), umfassend
- ein Halterunterteil (9) mit einem Aufnahmeraum (17) zur Aufnahme des Sensors (18), wobei
- der Aufnahmeraum (17) aufweist
-- einen Boden (19),
-- eine auf dem Boden (19) angeordnete, wenigstens an drei Seiten angeordnete Wandung (16),
-- eine durch wenigstens einen Steg (21, 22) gebildete Öffnung (20),
-- wenigstens einen Clipverschluss (23, 24), und
- weiterhin umfassend einen Deckel (10) für das Halterunterteil (9),
-- an dessen Innenseite (11) eine wenigstens dreiseitige Wandung (27) angeordnet ist,
-- deren Stirnseite (30) zur Auflage auf der Stirnseite (41) der Wandung (16) des Halterunterteils (9) ausgestaltet ist,
-- wobei an der Wandung (27) der Innenseite (11) des Deckels (10) wenigstens ein Gegenstück (28, 29) zu dem wenigstens einen Clipverschluss (23, 24) angeordnet ist, und
- das Halterunterteil (9) sowie der Deckel (10) durch ein Verbindungselement (13) derart miteinander verbunden sind, dass der Deckel (10) zum Halterunterteil (9) bewegbar ist, und
- das Halterunterteil (9) und der Deckel (10) über wenigstens einen Clipverschluss (23, 24) sowie das wenigstens eine Gegenstück (28, 29) gegeneinander arretierbar und zu öffnen sind.

2. Halterung nach Anspruch 1 **dadurch gekennzeichnet, dass**
- Verbindungselement (13) als Filmscharnier ausgestaltet ist.

3. Halterung nach Anspruch 1 oder 2 **dadurch gekennzeichnet, dass**
- an der Wandung (16) des Halterunterteils (9) eine Öffnung (20) an der proximalen Seite (14) des Halterunterteils (9) zur Aufnahme eines Sensorkabels (5) oder dessen Kabelschutzumhüllung (40) des Sensors (18) angeordnet ist.

4. Halterung nach Anspruch 3 **dadurch gekennzeichnet, dass**
- die Öffnung (20) durch den wenigstens einen von der Wandung (16) des Halterunterteils (9) abstehenden Steg (21, 22) flaschenhalsförmig verengt gegenüber dem Abstand der gegenüberliegenden Seiten der Wandung (16) des Halterunterteils (9) ist.

5. Halterung nach einem oder mehreren der vorstehenden Ansprüche **dadurch gekennzeichnet, dass**
- die Öffnung (20) der Wandung (16) des Halterunterteils (9) dergestalt verengt ist, dass der Sensor (18) nur durch Einlegen in den Aufnahmeraum (17) angeordnet werden kann.

6. Halterung nach einem oder mehreren der vorstehenden Ansprüche **dadurch gekennzeichnet, dass**
- der wenigstens eine Clipverschluss (23, 24) von der oberen Stirnseite (41) der Wandung (16) des Halterunterteils (9) oder des Stegs (21, 22) axial oder radial absteht.

7. Halterung nach einem oder mehreren der vorstehenden Ansprüche **dadurch gekennzeichnet, dass**
- der wenigstens eine Clipverschluss (23, 24) eine Verrundung zur Vermeidung von Spannungsspitzen beim Arretieren und/oder Öffnen des Deckels (10) aufweist.

8. Halterung nach einem oder mehreren der vorstehenden Ansprüche **dadurch gekennzeichnet, dass**
- die obere Stirnseite (41) der Wandung (16) des Halterunterteils (9) einen Rücksprung (25) aufweist.

9. Halterung nach einem oder mehreren der vorstehenden Ansprüche **dadurch gekennzeichnet, dass**
- die Stirnseite (30) der Wandung (27) der Innenseite (11) des Deckels (10) beim Arretieren des Deckels (10) mit dem Halterunterteil (9) auf der oberen Stirnseite (42) des Rücksprungs (25) der Wandung (16) des Halterunterteils (9) aufliegt.

10. Halterung nach einem oder mehreren der vorstehenden Ansprüche **dadurch gekennzeichnet, dass**
- der Boden (19) des Halterunterteils (9) wenigstens bereichsweise einen Randbereich (43) aufweist, der über die Auflagefläche der Wandung (16) des Halterunterteils (9) radial nach außen gerichtet ist.

11. Halterung nach einem oder mehreren der vorstehenden Ansprüche **dadurch gekennzeichnet, dass**
- der Deckel (10) wenigstens bereichsweise einen Randbereich (44) aufweist, der über die Auflagefläche der Wandung (27) der Innenseite (11) des Deckels (10) radial nach außen gerichtet ist.

12. Halterung nach einem oder mehreren der vorstehenden Ansprüche **dadurch gekennzeichnet, dass**
- der Deckel (10) einen eine bewegliche Zunge (31) aufnehmenden Ausschnitt (45) aufweist.

13. Halterung nach Anspruch 12 **dadurch gekennzeichnet, dass**
- die bewegliche Zunge (31) eine im geschlossenen Zustand des Halters (8) nach innen gerichtete Verdickung (32) zur Auflage auf die Oberseite (46) des Sensors (18) aufweist.

14. Halterung nach einem oder mehreren der vorstehenden Ansprüche **dadurch gekennzeichnet, dass**
- der Deckel (10) eine Ausnehmung (33) aufweist, die im geschlossenen Zustand der Halterung (8) an deren proximalen Ende liegt.

15. Halterung nach einem oder mehreren der vorstehenden Ansprüche **dadurch gekennzeichnet, dass**
- die Halterung als Einwegprodukt ausgestaltet ist.

16. Sensoranordnung (47), umfassend
- eine Halterung (8) nach einem oder mehreren der vorstehenden Ansprüche 1 - 15,
- einen wiederverwendbaren Sensor (18) zum Empfang elektrischer Ströme und/oder Signale in Bezug auf einen Körper eines Lebewesens und zur Übertragung dieser elektrischen Ströme und/oder Signale an ein Informations- und Darstellungssystem,
- eine Klebeanordnung (7),
- einen Isolator (6) aus einem Polyestergewebe oder Baumwollgewebe,
- eine Elektrode (4),
- ein Hydrogel (3).

17. Sensoranordnung nach Anspruch 16 **dadurch gekennzeichnet, dass**
- die Elektrode ein leitfähiges, mit Carbon versetztes Polymer aufweist.

18. Sensoranordnung nach Anspruch 16 oder 17 **dadurch gekennzeichnet, dass**
- der Sensor (18) ein Magnetsensor oder ein magnetoelastischer Sensor ist.

19. Sensoranordnung nach einem oder mehreren der Ansprüche 16 - 18 **dadurch gekennzeichnet, dass**
- die Sensoranordnung (47) und/oder der Isolator (6) schaumstofffrei ausgestaltet ist.

20. Sensoranordnung nach einem oder mehreren der vorstehenden Ansprüche 16 - 19 **dadurch gekennzeichnet, dass**
- der Sensor (18) kabellos oder über ein Kabel mit dem Informations- und Darstellungssystem verbunden ist.

21. Verwendung einer Halterung (8) nach einem oder mehreren der Ansprüche 1 - 15 als Reference Patch, aufweisend
- eine Klebeanordnung (7),
- einen schaumstofffreien Isolator (6) aus einem Kunstfasergewebe oder Baumwollgewebe,
- eine Elektrode (4),
- ein Hydrogel (3) und
- - mit einem Kabel (5).

22. Verwendung nach Anspruch 21 **dadurch gekennzeichnet, dass** das Sensorkabel (5) zwischen der Unterseite des Isolators (6) und der Oberseite der Elektrode (4) angeordnet ist.

## Claims

1. A mounting element (8) for releasably receiving a sensor (18) for transferring and/or receiving electrical currents and/or signals relating to a body of an organism, comprising
- a mounting element base (9) having a receiving space (17) for receiving the sensor (18),
- the receiving space (17) comprising
-- a floor (19),
-- a wall (16) disposed on the floor (19) and disposed on at least three sides,
-- an opening (20) formed by at least one tab (21, 22),
-- at least one clip closure (23, 24), and
- further comprising a cover (10) for the mounting element base (9),
-- an at least three-sided wall (27) being disposed on the inner side (11) thereof,
-- the end face (30) thereof being implemented for contacting the end face (41) of the wall (16) of the mounting element base (9),
-- wherein at least one counterpart (28, 29) to the at least one clip closure (23, 24) is disposed on the wall (27) of the inner side (11) of the cover (10), and
- the mounting element base (9) and the cover (10) are connected to each other by a connecting element (13) such that the cover (10) is displaceable relative to the mounting element base (9), and
- the mounting element base (9) and the cover (10) are therefore lockable to each other by means of the at least one clip closure (23, 24) and the at least one counterpart (28, 29) and can also be opened again.

2. The mounting element according to claim 1, **characterized in that**
- the connecting element (13) is implemented as an integral hinge.

3. The mounting element according to claim 1 or 2, **characterized in that**
- the wall (16) of the mounting element base (9) comprises an opening (20) for receiving a sensor cable (5) or a protective cable sheath (40) of the sensor (18) disposed at the proximal side (14) of the mounting element base (9).

4. The mounting element according to claim 3, **characterized in that**
- the opening (20) is narrowed in a bottleneck shape relative to the spacing of the opposite sides of the wall (16) of the mounting element base (9) by the at least one tab (21, 22) protruding from the wall (16) of the mounting element base (9).

5. The mounting element according to any one or more of the preceding claims, **characterized in that**
- the opening (20) of the wall (16) of the mounting element base (9) is narrowed such that the sensor (18) can be disposed only by placing in the receiving space (17).

6. The mounting element according to any one or more of the preceding claims, **characterized in that**
- the at least one clip closure (23, 24) is axially or radially spaced apart from the top end face (41) of the wall (16) of the mounting element base (9) or the tab (21, 22).

7. The mounting element according to any one or more of the preceding claims, **characterized in that**
- the at least one clip closure (23, 24) comprises a radius for preventing stress concentrations when locking and/or opening the cover (10).

8. The mounting element according to any one or more of the preceding claims, **characterized in that**
- the top end face (41) of the wall (16) of the mounting element base (9) comprises a recess (25).

9. The mounting element according to any one or more of the preceding claims, **characterized in that**
- the end face (30) of the wall (27) of the inner side (11) of the cover (10) makes contact with the mounting element base (9) at the top end face (42) of the recess (25) of the wall (16) of the mounting element base (9) when locking the cover (10).

10. The mounting element according to any one or more of the preceding claims, **characterized in that**
- the floor (19) of the mounting element base (9) comprises at least one edge region (43) at least in regions facing radially outward across the contact face of the wall (16) of the mounting element base (9).

11. The mounting element according to any one or more of the preceding claims, **characterized in that**
- the cover (10) comprises at least one edge region (44) at least in regions facing radially outward across the contact face of the wall (27) of the inner side (11) of the cover (10).

12. The mounting element according to any one or more of the preceding claims, **characterized in that**
- the cover (10) preferably comprises a cutout (45) for receiving a displaceable tongue (31).

13. The mounting element according to claim 12, **characterized in that**
- the displaceable tongue (31) comprises a thick part (32) facing inward in the closed state of the mounting element (8) for contacting the top side (46) of the sensor (18).

14. The mounting element according to any one or more of the preceding claims, **characterized in that**
- the cover (10) comprises a recess (33) at the proximal end of the mounting element (8) in the closed state thereof.

15. The mounting element according to any one or more of the preceding claims, **characterized in that**
- the mounting element is implemented as a disposable product.

16. A sensor arrangement (47), comprising
- a mounting element (8) according to any one or more of the preceding claims 1-15,
- a reusable sensor (18) for receiving electrical currents and/or signals with respect to a body of an organism and for transferring said electrical currents and/or signals to an information and imaging system,
- an adhesive arrangement (7),
- an insulator (6) made of a polyester fabric or cotton fabric,
- an electrode (4),
- a hydrogel (3).

17. The sensor arrangement according to claim 16, **characterized in that**
- the electrode comprises a conductive polymer infused with carbon.

18. The sensor arrangement according to claim 16 or 17, **characterized in that**
- the sensor (18) is a magnetic sensor or a magnetoelastic sensor.

19. The sensor arrangement according to any one or more of the claims 16-18, **characterized in that**
- the sensor arrangement (47) and/or the insulator (6) are implemented free of foam.

20. The sensor arrangement according to any one or more of the preceding claims 16-19, **characterized in that**
- the sensor (18) is connected to the information and imaging system wirelessly or by means of a cable.

21. A use of a mounting element (8) according to any one or more of the claims 1-15 as a reference patch, comprising
- an adhesive arrangement (7),
- a foam-free insulator (6) made of a synthetic fiber fabric or cotton fabric,
- an electrode (4),
- a hydrogel (3), and
- - having a cable (5).

22. The use according to claim 21, **characterized in that** the sensor cable (5) is disposed between the bottom side of the insulator (6) and the top side of the electrode (4).

## Revendications

1. Support de fixation (8) pour accueillir de manière amovible un capteur (18) servant à transmettre et/ou à recevoir des courants et/ou des signaux électriques en relation avec un corps d'un organisme vivant, comprenant
- une partie inférieure (9) de support de fixation avec un compartiment d'accueil (17) pour accueillir le capteur 18), dans lequel
- le compartiment d'accueil (17) présente
- un fond (19),
- une paroi (16) disposée sur le fond (19), au moins sur trois côtés de ce dernier,
- une ouverture (20) formée par au moins une bride (21, 22),
- au moins un fermoir à clip (23, 24) et
- comprenant en outre un couvercle (10) pour la partie inférieure (9) de support de fixation,
- sur la face intérieure (11) duquel est disposée une paroi (27) à au moins trois côtés,
- dont la face avant (30) se présente sous une forme pour s'appuyer contre la face avant (41) de la paroi (16) de la partie inférieure (9) de support de fixation,
- dans lequel au moins une contre-pièce (28, 29) de l'au moins un fermoir à clip (23, 24) est disposée sur la paroi (27) de la face intérieure (11) du couvercle (10), et
- la partie inférieure (9) de support de fixation ainsi que le couvercle (10) sont reliés l'un à l'autre par un élément de connexion (13) de sorte que le couvercle (10) soit déplaçable vers la partie inférieure (9) de support de fixation, et
- la partie inférieure (9) de support de fixation et le couvercle (10) peuvent être verrouillés et ouverts l'un par rapport à l'autre au moyen d'au moins un fermoir à clip (23, 24) ainsi que de l'au moins une contre-pièce (28, 29).

2. Support de fixation selon la revendication 1, **caractérisé en ce que**
- l'élément de connexion (13) se présente sous la forme d'une charnière à film.

3. Support de fixation selon la revendication 1 ou 2, **caractérisé en ce que**
- la paroi (16) de la partie inférieure (9) de support de fixation présente une ouverture (20) sur le côté proximal (14) de la partie inférieure (9) de support de fixation pour accueillir un câble (5) de capteur ou sa gaine de protection (40) de câble du capteur (18).

4. Support de fixation selon la revendication 3, **caractérisé en ce que**
- l'ouverture (20) est rétrécie en forme de goulot de bouteille par l'au moins une bride (21, 22) faisant saillie de la paroi (16) de la partie inférieure (9) de support de fixation par rapport à la distance entre les côtés opposés de la paroi (16) de la partie inférieure (9) de support.

5. Support de fixation selon l'une quelconque ou plusieurs des revendications précédentes, **caractérisé en ce que**
- l'ouverture (20) de la paroi (16) de la partie inférieure (9) de support de fixation est rétrécie de sorte que
le capteur (18) ne puisse être mis en place que par insertion dans le compartiment d'accueil (17).

6. Support de fixation selon l'une quelconque ou plusieurs des revendications précédentes, **caractérisé en ce que**
- l'au moins un fermoir à clip (23, 24) fait saillie axialement ou radialement de la face avant supérieure (41) de la paroi (16) de la partie inférieure (9) de support de fixation ou de la bride (21 22).

7. Support de fixation selon l'une quelconque ou plusieurs des revendications précédentes, **caractérisé en ce que**
- l'au moins un fermoir à clip (23, 24) présente un arrondi pour éviter les crêtes de tension lors du verrouillage et/ou de l'ouverture du couvercle (10).

8. Support de fixation selon l'une quelconque ou plusieurs des revendications précédentes, **caractérisé en ce que**
- la face avant supérieure (41) de la paroi (16) de la partie inférieure (9) de support de fixation présente un retrait (25).

9. Support de fixation selon l'une quelconque ou plusieurs des revendications précédentes, **caractérisé en ce que**
- la face avant (30) de la paroi (27) de la face intérieure (11) du couvercle (10) repose sur la face avant supérieure (42) du retrait (25) de la paroi (16) de la partie inférieure (9) de support de fixation lors du verrouillage du couvercle (10) avec la partie inférieure (9) de support de fixation.

10. Support de fixation selon l'une quelconque ou plusieurs des revendications précédentes, **caractérisé en ce que**
- le fond (19) de la partie inférieure (9) de support de fixation présente au moins dans certaines régions une zone de bordure (43) qui est dirigée radialement vers l'extérieur sur la surface d'appui de la paroi (16) de la partie inférieure (9) de support de fixation.

11. Support de fixation selon l'une quelconque ou plusieurs des revendications précédentes, **caractérisé en ce que**
- le couvercle (10) présente au moins dans certaines régions une zone de bordure (44) qui est dirigée radialement vers l'extérieur sur la surface d'appui de la paroi (27) de la face inférieure (11) du couvercle (10).

12. Support de fixation selon l'une quelconque ou plusieurs des revendications précédentes, **caractérisé en ce que**
- le couvercle (10) présente une encoche (45) accueillant une languette mobile (31).

13. Support de fixation selon la revendication 12, **caractérisé en ce que**
- la languette mobile (31) présente un épaississement (32) qui, à l'état fermé du support de fixation (8), est dirigé vers l'intérieur pour s'appuyer sur la face supérieure (46) du capteur (18).

14. Support de fixation selon l'une quelconque ou plusieurs des revendications précédentes, **caractérisé en ce que**
- le couvercle (10) présente un évidement (33) qui, à l'état fermé du support (8) de fixation, est disposé à l'extrémité proximale de ce dernier.

15. Support de fixation selon l'une quelconque ou plusieurs des revendications précédentes, **caractérisé en ce que**
- le support de fixation se présente sous la forme d'un produit à usage unique.

16. Agencement de capteur (47), comprenant
- un support de fixation (8) selon l'une quelconque ou plusieurs des revendications 1 - 15 précédentes,
- un capteur réutilisable (18) servant à recevoir des courants et/ou des signaux électriques en relation avec un corps d'un organisme vivant et à transmettre ces courants et/ou signaux électriques à un système d'information et d'affichage,
- un composant adhésif (7),
- un isolateur (6) en un tissu de polyester ou de coton,
- une électrode (4),
- un hydrogel (3).

17. Agencement de capteur selon la revendication 16, **caractérisé en ce que**
- l'électrode présente un polymère conducteur auquel est ajouté du carbone.

18. Agencement de capteur selon la revendication 16 ou 17, **caractérisé en ce que**
- le capteur (18) est un capteur magnétique ou un capteur magnéto-élastique.

19. Agencement de capteur selon l'une quelconque ou plusieurs des revendications 16 - 18 précédentes, **caractérisé en ce que**
- l'agencement de capteur (47) et/ou l'isolateur (6) se présente(nt) sous une forme exempte de mousse.

20. Agencement de capteur selon l'une quelconque ou plusieurs des revendications 16 - 19 précédentes, **caractérisé en ce que**
- le capteur (18) est connecté au système d'information et d'affichage de manière sans câble ou via un câble.

21. Utilisation d'un support de fixation (8) selon l'une quelconque ou plusieurs des revendications 1 - 15 en tant que Reference Patch, comprenant
- un composant adhésif (7),
- un isolateur (6) exempt de mousse en un tissu de fibres synthétiques ou tissu de coton,
- une électrode (4),
- un hydrogel (3) et
- avec un câble (5).

22. Utilisation selon la revendication 21, **caractérisée en ce que** le câble (5) de capteur est disposé entre la face inférieure de l'isolateur (6) et la face supérieure de l'électrode (4).
